Europäisches Patentamt

European Patent Office

Office européen des brevets

(19)

(11) **EP 0 547 932 B1**

(12) **FASCICULE DE BREVET EUROPEEN**

(45) Date de publication et mention
de la délivrance du brevet:
**04.09.1996 Bulletin 1996/36**

(51) Int Cl.[6]: **C12N 9/64**
**// A61K38/48**

(21) Numéro de dépôt: **92403234.5**

(22) Date de dépôt: **01.12.1992**

(54) **Procédé de fabrication d'un concentré de facteur VII activé de haute pureté**

Verfahren zur Herstellung eines aktivierten Faktor VII-Konzentrates mit einer hohen Reinheit

Process for preparing an activated factor VII concentrate having a high purity

(84) Etats contractants désignés:
**AT BE CH DE DK ES FR GB GR IE IT LI LU MC NL PT SE**

(30) Priorité: **16.12.1991 FR 9115601**

(43) Date de publication de la demande:
**23.06.1993 Bulletin 1993/25**

(73) Titulaire: **ASSOCIATION D'AQUITAINE POUR LE DEVELOPPEMENT DE LA TRANSFUSION SANGUINE ET DES RECHERCHES HEMATOLOGIQUES**
**F-33035 Bordeaux Cédex (FR)**

(72) Inventeurs:
• **Dazey, Bernard**
  **F-33000 Bordeaux (FR)**
• **Hamsany, Mohamed**
  **F-33000 Bordeaux (FR)**
• **Enfedaque-Morer, Sylvia**
  **F-33000 Bordeaux (FR)**

(74) Mandataire: **Portal, Gérard et al**
**Cabinet Beau de Loménie**
**158, rue de l'Université**
**75340 Paris Cédex 07 (FR)**

(56) Documents cités:
**EP-A- 0 346 241        US-A- 4 473 553**

• **RESEARCH DISCLOSURE, vol. 269, septembre 1986, Havant (GB); S. BJOERN et al., pp. 564-565**

## Description

La présente invention concerne essentiellement le procédé de fabrication d'un concentré de facteur VII activé de haute pureté essentiellement dépourvu des facteurs vitamine K dépendants et des facteurs VIIIC et VIIICAg, ainsi que le produit obtenu par ce procédé.

Le facteur de la coagulation responsable du rétablissement de l'hémostase chez les hémophiles ayant des anticorps circulants est connu comme étant le facteur VII activé. Celui-ci rentre dans La composition de concentrés prothrombiniques activés comme par exemple le FEIBA® ou l'AUTOPLEX®.

Différents documents tels que WO-A-89/12097, WO-A-83/00016, EP-A-0 225 160, US-A-4 637 932 et FR-A-2 622 108 ont mis en évidence le rôle effectif du facteur VIIa. En particulier, dans le document EP-B1-0 225 160, il est démontré l'efficacité du facteur VIIa dans le rétablissement d'une hémostase normale dans le cas de désordres hémorragiques non provoqués par des déficiences en facteur VIII ou par des inhibiteurs.

Ces documents, ainsi que d'autres, décrivent différentes techniques de purification du facteur VII et du facteur VIIa. Cependant, les techniques utilisées sont très complexes, nécessitent de nombreuses étapes et sont difficilement applicables à l'échelle industrielle et médicale, ou nécessitent un coût élevé.

Par exemple, PANCHAM décrit dans US-A-4 637 932 un procédé de purification de facteurs VII et de facteurs VIIa à partir d'une solution aqueuse contenant des protéines plasmatiques par adsorption de ces protéines sur un gel portant des sels de métal divalent ayant une affinité pour les protéines se fixant au calcium, tels que le phosphate tricalcique sous forme d'hydroxyapatite de la Société Biorad® que l'on désorbe ensùite à l'aide d'un tampon contenant des sels capables de déplacer les protéines fixées.

Une purification complémentaire est ensuite réalisée à l'aide d'une chromatographie d'échange d'ions type DEAE (DiEthyl-AminoEthyle) par exemple disponible dans le commerce sous la dénomination commerciale DEAE Sephadex® selon la procédure décrite dans US-A-3 717 708.

Le document RESEARCH DISCLOSURE vol. 269, septembre 1986, HAVANT GB, pages 564-565, contient un article de BJOERN relatif à l'activation du facteur VII de coagulation en facteur VIIa. La méthode décrite consiste à réaliser une adsorption sélective sur une colonne d'échange ionique type DEAE-SEPHADEX, QAE-SEPHADEX ou Mono Q de chez PHARMACIA, du facteur VII pour réaliser son activation. Il est à noter que cette méthode n'apparaît être valable qu'à partir d'un facteur VII précédemment purifié par des techniques classiques ou obtenu par les méthodes du génie-génétique,' comme cela est dit à la page 564.

Par contre, dans le cadre de l'invention, on recherche à activer du facteur VII non purifié présent dans le plasma.

Le document EP-A 0 346 241 de la Fondation Nationale de Transfusion Sanguine ou FNTS = WO-A-89/12097 = FR-A-2 632 524 décrit un procédé de préparation d'une fraction riche en facteur VIIa utilisant une étape de fixation au calcium d'un prééluat du PPSB après ajout d'un inhibiteur de la kallikréine, tel que l'aprotinine qui introduit une protéine étrangère pouvant avoir un caractère contaminant. La première étape d'activation est réalisée avec du phosphate tricalcique pour lequel le facteur VII a une grande affinité, puis on réalise des étapes de désorption par des sels capables de reséparer les protéines fixées au calcium, comme le phosphate mono ou disodique, l'activation du facteur VII est complétée par une activation au kaolin ou à la célite. Avant l'inactivation virale, on rajoute de l'antithrombine III pour éviter toute précipitation prématurée (colonne 5, exemple 2, lignes 31 à 36). Enfin, on réalise une inactivation virale, puis enfin on élimine les agents d'inactivation par chromatographie sur colonne de Q-Sépharose. Enfin, pour stabiliser les protéines on rajoute de l'albumine (exemple 2, colonne 5, lignes 45-47). Ainsi, ce procédé est compliqué, nécessite en cours de procédure de vérifier l'activation, et encore d'ajouter trois protéines étrangères, à savoir l'aprotinine, l'antithrombine III et de l'albumine, ce qui peut avoir un risque potentiel de contamination. Enfin, le facteur VII obtenu contient encore des quantités non négligeables d'autres facteurs de coagulation, comme cela résulte du tableau en colonne 6, notamment le facteur II, le facteur IX et le facteur X, ce qui démontre le risque thrombogénique.

Le document US-A-4 473 553 ZUFFI parle tout d'abord d'une fraction dite de Cohn qui résulte d'un ou plusieurs relargages à l'alcool du plasma initial. Cette fraction de Cohn est ensuite traitée avec un mélanae contenant en majorité très grande du chlorure de calcium avec une faible quantité d'un adsorbant polyanionique pouvant être du sulfate de dextran (exemple 1), ou de l'héparine (exemple 2), afin d'éliminer les lipoprotéines par précipitation. Dans ce mélange, le sulfate de dextran n'a pas d'effet activant détectable. L'activation du facteur VII se produit ultérieurement avec de l'hydroxyapatite.

L'invention diffère de ce document en utilisant directement le plasma qui est activé directement seulement par des billes de sulfate de dextran sans précipitation de lipoprotéine à ce stade, ce qui simplifie ensuite le procédé et procure un très bon taux d'activation.

D'autre part, MICHALSKY et al. dans FR-A-2 622 108 décrivent un procédé de purification du facteur VII par chromatographie d'adsorption sur DEAE Sepharose® suivie d'une élution avec une solution tampon à 0,18-0,20 M de NaCl qui conduit à l'obtention d'un facteur VII ayant une activité spécifique comprise entre 0,8 et 1, ce qui constitue un très faible taux de purification.

Enfin, dans l'article de HEDNER et KIESEL dans J. Clin. (1983), 71, p. 1836-1841, on décrit l'utilisation de facteur VII activé humain dans le traitement de deux patients souffrant d'hémophilie A avec une forte proportion d'inhibiteurs. Dans leur traitement, ces auteurs utilisent comme facteur VII activé, un facteur VII qui a été purifié par chromatographie d'échange d'ions, (voir p. 1837 colonne de gauche), à l'aide d'une colonne DEAE-Sepharose® en éluant avec des solutions tampons contenant du chlorure de calcium. Le facteur VII est activé par emploi de facteur XII activé lui-même obtenu grâce à une activation par la kallikréine humaine en présence de sulfate de dextran. Cette procédure est également complexe et exige l'apport de protéines éxogénes, dont le facteur XII activé et la kallikréine, qu'il est nécessaire d'éliminer en fin d'étape de purification pour obtenir un facteur VII activé de très haute pureté.

La présente invention a donc pour but principal de résoudre le nouveau problème technique consistant en la fourniture d'une solution permettant de fabriquer du facteur VII activé de haute pureté, essentiellement dépourvu des facteurs vitamine K dépendants et des facteurs VIIIC et VIIICAg.

La présente invention a encore pour but de résoudre ce nouveau problème technique par l'emploi d'une solution n'impliquant pas l'emploi de protéines exogènes pour l'activation du facteur VII.

La présente invention a encore pour but de résoudre le nouveau problème technique énoncé ci-dessus avec une solution simple, notamment dans l'étape d'activation du facteur VII, et également dans les étapes de purification proprement dites.

La présente invention résout pour la première fois ce problème technique énoncé ci-dessus d'une manière simple, relativement peu coûteuse, utilisable à l'échelle industrielle et médicale tout en aboutissant à un facteur VII activé de haute pureté essentiellement dépourvu des facteurs vitamine K dépendants et des facteurs VIIIC et VIIICAg.

Ainsi, selon un premier aspect, la présente invention fournit un procédé de fabrication d'un concentré de facteur VII activé de haute pureté comprenant l'emploi de plasma dépourvu de cryoprécipité, de préférence d'origine humaine, ainsi qu'au moins une étape de purification par au moins une chromatographie sur résine d'échange d'anions ainsi qu'une étape d'activation du facteur VII, caractérisé en ce qu'on réalise tout d'abord l'activation directe du facteur VII dans le plasma dépourvu de cryoprécipité, sans apport de protéines éxogénes par emploi de sulfate de dextran sous forme pulvérulente, insoluble dans ledit plasma. De préférence, le sulfate de dextran est utilisé sous forme de billes. La dimension des billes n'est pas critique et l'on pourra utiliser des billes de sulfate de dextran disponibles dans le commerce telles que les billes commercialisées par la société Sigma, référence D5650. Il est à noter qu'aucun traitement spécial de ces billes n'est nécessaire avant l'utilisation, ce qui constitue également un avantage important de l'invention.

Selon une autre caractéristique avantageuse du procédé selon l'invention, l'étape d'activation du facteur VII, de préférence par le sulfate de dextran est réalisée à froid à une température contrôlée, avantageusement comprise entre 0°C et +4°C, ce qui permet d'assurer une très bonne activation du facteur VII en facteur VIIa.

Selon une autre caractéristique avantageuse du procédé selon l'invention, après l'étape d'activation précitée, on procède à une chromatographie d'échange d'anions de type DEAE du plasma dépourvu de cryoprécipité contenant le facteur VII activé ainsi que du facteur VII non activé, de manière à adsorber sélectivement essentiellement tous les facteurs vitamine K dépendants, c'est-à-dire comprenant principalement le facteur VII activé, le facteur VII non activé, le facteur IX, le facteur X et le facteur II. Ainsi, le but de cette adsorption sélective est d'éliminer une partie significative des protéines indésirables telles que l'albumine.

Selon encore une autre caractéristique avantageuse du procédé selon l'invention, on réalise une désorption non sélective de tous les facteurs vitamine K dépendants adsorbés sur la colonne d'échange d'ions DEAE précitée. Pour ce faire, on peut éluer avec toute solution tampon d'élution bien connue de l'homme de l'art pour réaliser cette désorption sur ce type de gel.

Ensuite, l'éluat obtenu contenant tous les facteurs vitamine K dépendants est à nouveau passé sur une colonne d'échange d'anions spécifique typa amine quaternaire, de préférence de type Q Sépharose Fast Flow® de chez PHARMACIA qui se présente sous forme de billes d'agarose, réticulée à environ 6 % et comprenant de préférence un petit bras espaceur en $C_1$-$C_6$, de manière à adsorber sélectivement tous les facteurs vitamine K dépendants.

Cette adsorption des facteurs vitamine K dépendants de manière spécifique sur cette colonne permet ainsi d'éliminer d'autres protéines contaminantes comme l'$\alpha$-antitrypsine et la céruloplasmine.

Cette adsorption sélective sur colonne type amine quaternaire constitue une caractéristique brevetable du procédé, indépendante de l'activation du facteur VII. Il en est de même pour la désorption sélective qui est mentionnée ci-après. De préférence, la solution d'adsorption sélective est une solution tampon NaCl 150 mM, citrate trisodique 4 mM et pH 6.

On procède ensuite à une désorption sélective du facteur VII activé et du facteur VII non activé en procédant à une élution de la colonne avec une solution tampon dite de désorption sélective, ayant la composition suivante :

- NaCl     250 mM
- citrate trisodique    4 mM
- pH 6

L'éluat obtenu contenant essentiellement le facteur VII activé et le facteur VII non activé sous forme aqueuse, essentiellement dépourvu des autres facteurs vitamine K dépendants, subit ensuite une étape d'inactivation virale de manière connue, par exemple par l'emploi d'un mélange de TNBP/Tween en observant la proportion relative de 0,3 % en TNBP et 1 % en Tween par rapport à la concentration totale de la solution de facteur VII activé/ facteur VII, de préférence pendant 6 h à 24°C sous agitation conformément au procédé décrit dans le document EP-A-0 131 740 ou USA 4 540 573.

Après inactivation, il est nécessaire d'éliminer le mélange d'inactivateurs TNBP/Tween résiduel et on procède à nouveau à une adsorption sur la même colonne précitée de Q Sépharose FAST FLOW® avec une solution tampon permettant l'adsorption sélective, à savoir NaCl 150 mM, citrate trisodique 4 mM et pH 6.

Cette adsorption est ensuite suivie de la désorption avec la même solution de désorption (NaCl 250 mM, citrate trisodique 4 mM, pH 6) en obtenant ainsi un éluat contenant le facteur VII activé de haute pureté, et le facteur VII non activé essentiellement dépourvu de facteurs vitamine K dépendants ainsi que des facteurs VIIIC et VIIICAg.

On peut ensuite encore diafiltrer cet éluat contre un tampon de préférence compatible à l'injection chez l'homme (tel qu'un tampon NaCl 100 mM, citrate trisodique 4 mM, lysine 3 g/l, pH 7) que l'on peut avantageusement concentrer à un taux de 5 000 UI en facteur VII activé par flacon de 20 ml que l'on peut filtrer stérilement par filtration sur un filtre stérilisant à une dimension de pores de 0,2 micromètre, puis procéder à une lyophilisation si cela est souhaité.

Il est à noter que le procédé selon l'invention permet non seulement d'obtenir un facteur VII de haute pureté, mais également un taux d'activation très élevé du facteur VII.

De ce fait, l'expression "facteur VII activé de haute pureté" signifie dans la description et dans les revendications que le rapport facteur VIIa/facteur VII est supérieur à 5, de préférence supérieur à 7 et encore de préférence voisin de 10, tandis que l'activité spécifique du facteur VII activé est supérieure à 200 UI/mg de protéine. L'activité thrombogénique est négative à 6 h à 37°C selon le test de la thrombine libre.

L'invention couvre également le facteur VII activé de haute pureté obtenu par le procédé précédemment décrit.

D'autres buts, caractéristiques et avantages de l'invention apparaîtront clairement à la lumière de la description explicative qui va suivre faite en référence à deux exemples de fabrication donnés simplement à titre d'illustration et qui ne sauraient donc en aucune façon limiter la portée de l'invention. Dans les exemples tous les pourcentages sont donnés en poids, sauf indication contraire.

Exemple 1

5 l de plasma dépourvu de cryoprécipité, maintenu à 4°C sont ajustés à une force ionique < à 140 mM en NaCl.

On ajoute, en batch, 0,1 g de billes de sulfate de dextran (Sigma D5650) par litre de plasma. Après 1 h d'agitation à 4°C, on décante et le surnageant est récupéré pour être incubé pendant 90 min avec 0,5 g/l de DEAE Sephadex A50®. Après décantation, le gel sur lequel se sont fixés tous les facteurs vitamine K dépendants est lavé avec 1 l de tampon citrate trisodique 4 mM, NaCl 140 mM, pH 7 qui permet d'éliminer entre autres l'albumine. Les facteurs vitamine K dépendants sont ensuite élués avec ce même tampon dont la force ionique a été augmentée à 500 mM en NaCl. On récupère 500 ml d'éluat I.

Cet éluat I est diafiltré et concentré à 250 ml avec une cassette d'un seuil de coupure de 10 Kd contre un tampon citrate trisodique 4 mM, NaCl 150 mM, pH 6.

Cette solution est chromatographiée sur une colonne de 9 cm de diamètre contenant 300 ml de gel Q Sépharose Fast Flow $^R$ (Pharmacia) équilibrée avec le même tampon. 5 l de ce tampon vont permettre d'éliminer l'$\alpha$-antitrypsine et la céruloplasmine. Les facteurs VII/VIIa vont être désorbés spécifiquement en augmentant la force ionique à 250 mM en NaCl, on récupère environ 2 l d'éluat II.

L'éluat II subit ensuite une étape d'inactivation virale par un mélange de TNBP/Tween tel que la concentration finale dans la solution de facteurs VII/VIIa soit de 0,3 % en TNBP et 1 % en Tween pendant 6 h à 24°C sous agitation.

Après inactivation, la solution est diafiltrée et concentrée à 250 ml contre le tampon servant à l'équilibration de la colonne de Q Sépharose FAST FLOW® précédemment citée (citrate trisodique 4 mM, NaCl 150 mM, pH 6). La solution est à nouveau chromatographiée sur Q Sépharose FAST FLOW ® avec 2 l de solution de lavage identique au tampon d'équilibration, ce qui permet d'éliminer le mélange d'inactivateurs en le faisant passer dans la fraction non retenue. Avec le tampon citrate trisodique 4 mM, NaCl 250 mM, pH 6, on élue sélectivement les facteurs VII/VIIa relativement aux autres facteurs vitamine K dépendants pour obtenir un éluat III. Cet éluat de 2 l est ensuite diafiltré contre un tampon compatible à l'injection chez l'homme (citrate trisodique 4 mM, NaCl 100 mM, lysine 3 g/l, pH 7) et concentré à un taux de 5 000 UI en facteur VIIa par flacon de 20 ml, puis lyophilisé.

Les résultats obtenus par le procédé selon l'invention à l'échelle pilote sur 4 expériences portant chacune sur 5 l de plasma dépourvu de cryoprécipité ainsi qu'à titre de comparaison sur un lot dit témoin selon le même procédé mais avec la suppression de l'étape d'activation en sulfate de dextran sont rapportés au tableau I.

Les résultats sont exprimés en unité internationale au tableau I. Les déterminations sont effectuées à divers stades du procédé comme cela est bien compréhensible à la considération du tableau I, pour l'homme de l'art.

TABLEAU I

|  | VII | VIIa | VIIa/VII | RdVII % | RdVIIa % | Lot témoin | | |
|---|---|---|---|---|---|---|---|---|
|  |  |  |  |  |  | VII | VIIa | VIIa/VII |
| PDC* | 4212 ± 318 | 7400 ± 1030 | 1,7 ± 0,3 | 100 | 100 | 3250 | 5000 | 2 |
| APRES SULFATE DEXTRAN | 5415 ± 616 | 35275 ± 3985 | 6,9 ± 1,3 | 127 ± 10 | 500 ± 81 |  |  |  |
| APRES DEAE | 4292 ± 253 | 54167 ± 9610 | 13 ± 2,8 | 99 ± 9 | 657 ± 93 | 1850 | 3600 | 2 |
| APRES Q1 | 1960 ± 390 | 26313 ± 3870 | 15,8 ± 3,3 | 44,7 ± 6,4 | 359 ± 31 | 1050 | 2030 | 2 |
| APRES Q2+LY0 | 1500 ± 244 | 17062 ± 4320 | 11,7 ± 2 | 33 ± 4,5 | 234 ± 54 | 940 | 1800 | 2 |

*PDC = plasma dépourvu de cryoprécipité
Q1 : 1$^{re}$ chromatographie d'adsorption sur Q Sepharose Fast Flow
Q2 : 2$^e$ chromatographie d'adsorption sur Q Sepharose Fast Flow
LYO : lyophilisation.

Caractéristiques biologiques du concentré de facteur VIIa après reconstitution par 20 ml d'eau distillée :

| | |
|---|---|
| VIIa : | 172 ± 27 UI/ml |
| VII : | 14,3 ± 1,4 UI/ml |
| VIIAg : | 0,88 ± 0,22 UI/ml |
| VIIa/VII : | 12 ± 2,5 |
| Taux de protéines : | 0,82 ± 0,07 |
| AS VIIa : | 208 ± 36 |
| AS VII : | 17,5 ± 1,6 |
| II/IIa : | <0,1 UI/ml |
| IX/IXa : | <0,1 UI/ml |
| X/Xa : | <0,1 UI/ml |
| VIIIC : | <0,1 UI/ml |
| VIIICAg : | <0,1 UI/ml |

Les dosages des facteurs de la coagulation se font par des mesures chronométriques sur le raccourcissement du temps de coagulation d'un plasma déficient pour un facteur manquant. Ce facteur sera apporté par l'échantillon.

Le degré d'activation du facteur VII est déterminé par l'utilisation de thromboplastine humaine et de thromboplastine bovine selon la méthode décrite par Van Deijk et al., Haemostasis 13,1983.

Le facteur VII activé a une affinité plus grande pour la thromboplastine bovine que le facteur VII, alors qu'ils ont la même affinité pour la thromboplastine humaine. Le degré d'activation est exprimé par le rapport :

$$\frac{\text{Facteur VII avec thromboplastine bovine}}{\text{Facteur VII avec la thromboplastine humaine}}$$

Par référence, ce rapport est de 1 dans un plasma humain normal.

Exemple 2 : Résultats obtenus sur un lot industriel de 300 l de plasma.

On procède comme décrit à l'exemple 1 si ce n'est que l'on utilise au départ 300 l de plasma.
Les résultats obtenus sont exprimés au tableau II ci-après.

TABLEAU II

|  | VIIa | VII | AS VIIa | AS VII |
|---|---|---|---|---|
| PDC | 391300 | 301154 | 0,034 | 0,022 |
| APRES SULFATE DE DEXTRAN | 2530000 | 361507 | 0,24 | 0,029 |
| APRES DEAE | 1282500 | 157800 | 8,3 | 1,02 |
| APRES Q1 | 1399650 | 150500 | 73 | 7,8 |

TABLEAU II   (suite)

| | VIIa | VII | AS VIIa | AS VII |
|---|---|---|---|---|
| APRES Q2 + LY0 | 965000 | 98800 | 244 | 32,5 |
| PDC, Q1, Q2 et LY0 : significations voir tableau I. | | | | |

Caractéristiques biologiques du concentré de facteur VIIa après reconstitution par 20 ml d'eau distillée :

VIIa : 244 UI/ml
VII : 32,5 UI/ml
VIIa/VII : 7,5
Taux de protéines : 1 g/l
AS VIIa : 244
AS VII : 32,5
II/IIa : <0,1 UI/ml
IX/IXa : <0,1 UI/ml
X/Xa : <0,1 UI/ml
VIIIC : <0,1 UI/ml
VIIICAg : <0,1 UI/ml

On peut ainsi observer à partir des exemples précédents et des tableaux exprimant les résultats qu'avec le lot témoin, sans activation initiale, le rapport VII/VIIa n'est sensiblement pas modifié puisqu'il est voisin du rapport initial de 1,5 d'un plasma normal.

Par ailleurs, l'invention permet, en plus du fait de l'obtention d'un très bon rapport d'activation, d'obtenir un excellent taux de purification du facteur VII avec un procédé extrêmement simple, aisé à mettre en oeuvre, avec une très bonne reproductibilité.

Par ailleurs, le produit selon l'invention présente une trés faible activité thrombogénique. En effet, le test de la thrombine libre (PHARMACOPEE Européenne 2e édition, 554) est négatif à 6 h et à 37°C avec le produit de l'invention, ce qui constitue une caractéristique essentielle préférée du produit de l'invention qui le distingue des produits de l'art antérieur. En effet, les concentrés de facteur VII activés obtenus par d'autres procédés ainsi que les concentrés prothrombiniques activés de la technique antérieure comme par exemple le FEIBA® ou l'AUTOPLEX® ont une activité thrombogénique positive avant 6 h.

Il est également à noter que le concentré de facteur VII activé selon l'invention n'est pas traité à l'antithrombine III en raison de sa pureté et ne présente donc aucun risque de coagulation prématurée.

Par ailleurs, notamment en vue de la lyophilisation, le concentré de facteur VII activé selon la présente invention, en raison de sa pureté, ne nécessite pour sa stabilisation que l'emploi de lysine alors que les produits antérieurs nécessitaient pour la lyophilisation l'emploi d'albumine, ce qui diminue le coût et évite d'injecter une protéine inutile pour le patient.

**Revendications**

1. Procédé de fabrication d'un concentré de facteur VII activé de haute pureté comprenant l'emploi d'un plasma dépourvu de cryoprécipité, de préférence d'origine humaine, ainsi qu'au moins une étape de purification par au moins une chromatographie sur résine d'échange d'anions ainsi qu'une étape d'activation du facteur VII, caractérisé en ce qu'on réalise tout d'abord l'activation directe du facteur VII dans le plasma dépourvu de cryoprécipité, sans apport de protéines exogènes par l'emploi de sulfate de dextran sous forme pulvérulente, insoluble dans ledit plasma.

2. Procédé selon la revendication 1, caractérisé en ce que le sulfate de dextran est utilisé sous forme de billes.

3. Procédé selon l'une des revendications 1 à 2, caractérisé en ce que l'étape d'activation du facteur VII, de préférence par le sulfate de dextran, est réalisée à froid à une température contrôlée, avantageusement comprise entre 0°C et +4°C.

4. Procédé selon l'une des revendications 1 à 3, caractérisé en ce que, après l'étape d'activation précitée, on procède à une chromatographie d'échange d'anions de type DEAE, du plasma dépourvu de cryoprécipité contenant le facteur VII activé ainsi que du facteur VII non activé, de manière à adsorber sélectivement tous les facteurs vitamine K dépendants.

5.  Procédé selon la revendication 4, caractérisé en ce qu'on réalise une désorption non sélective de tous les facteurs vitamine K dépendants adsorbés sur la colonne d'échange d'ions DEAE précitée.

6.  Procédé selon la revendication 5, caractérisé en ce que l'éluat obtenu contenant les facteurs vitamine K dépendants est à nouveau passé sur une colonne d'échange d'anions spécifiques type amine quaternaire, de préférence de type Q Sepharose Fast Flow® de chez PHARMACIA, qui se présente sous forme de billes d'agarose réticulées à environ 6 %, et comprenant de préférence un petit bras espaceur en $C_1$-$C_6$, de manière à adsorber sélectivement tous les facteurs vitamine K dépendants.

7.  Procédé selon la revendication 6, caractérisé en ce qu'on procède ensuite à une désorption sélective du facteur VII activé du facteur VII non activé en procédant à une élution de la colonne avec une solution tampon dite de désorption sélective, ayant la composition suivante :

    -   Nacl        250 mM
    -   citrate trisodique         4 mM
    -   pH 6

8.  Procédé selon la revendication 7, caractérisé en ce que l'éluat obtenu contenant essentiellement le facteur VII activé et le facteur VII non activé résiduel sous forme aqueuse, essentiellement dépourvu des autres facteurs vitamine K dépendants, subit ensuite une étape d'inactivation virale, par exemple par l'emploi d'un mélange de TNBP/Tween en observant la proportion relative de 0,3 % en TNBP et 1 % en Tween par rapport à la concentration totale de la solution de facteur VII activé/facteur VII, de préférence pendant 6 h à 24°C sous agitation, le mélange d'inactivation est ensuite éliminé par adsorption sur la même colonne avec la même solution tampon permettant l'adsorption sélective (NaCl 150 mM, citrate trisodique 4 mM et pH 6), avec désorption sélective avec la même solution tampon de désorption sélective précitée.

9.  Procédé selon la revendication 8, caractérisé en ce qu'on réalise une diafiltration comprenant un tampon, de préférence compatible à l'injection chez l'homme tel qu'un tampon NaCl 100 mM, citrate trisodique 4 mM, lysine 3 g/l, pH 7.

10. Procédé selon la revendication 9, caractérisé en ce qu'on réalise une concentration à un taux de 5 000 UI en facteur VIIa activé par flacon de 20 ml Que l'on peut filtrer stérilement par filtration sur un filtre stérilisant à une dimension de pores de 0,2 micromètre, puis éventuellement on procède à une lyophilisation.

## Claims

1.  Process for the manufacture of a high-purity activated factor VII concentrate comprising the use of a cryoprecipitate-free plasma, preferably of human origin, as well as at least one purification stage by chromatographing at least once an anion exchange resin, as well as an activation stage of the factor VII, characterized in that direct activation of the factor VII is carried out first of all in the cyroprecipitate-free plasma, without introduction of exogenous proteins, by using dextran sulphate in the pulverulent form which is insoluble in the said plasma.

2.  Process according to claim 1, characterized in that dextran sulphate is used in the form of beads.

3.  Process according to one of claims 1 to 2, characterized in that the activation stage of the factor VII, preferably by dextran sulphate, is carried out while cold at a controlled temperature, advantageously between 0°C and +4°C.

4.  Process according to one of claims 1 to 3, characterized in that, after the above-mentioned activation stage, DEAE-type anion exchange chromatography of the cyroprecipitate-free plasma containing the activated factor VII as well as the unactivated factor VII is carried out so as to selectively adsorb all the vitamin K-dependent factors.

5.  Process according to claim 4, characterized in that non-selective desorption of all the vitamin K-dependent factors adsorbed on the above-mentioned DEAE ion exchange column is carried out.

6. Process according to claim 5, characterized in that the eluate obtained containing the vitamin K-dependent factors is again passed through a specific anion exchange column of quaternary amine type, preferably Q Sepharose Fast Flow® type from PHARMACIA, which is provided in the form of agarose beads cross-linked to the extent of approximately 6% and preferably comprising a small $C_1$-$C_6$ spacer arm, so as to selectively adsorb all the vitamin K-dependent factors.

7. Process according to claim 6, characterized in that selective desorption of the activated factor VII and of the unactivated factor VII is then carried out by eluting the column with a buffer solution, known as selective desorption, having the following composition:

   - NaCl 250 mM
   - trisodium citrate 4mM
   - pH 6

8. Process according to claim 7, characterized in that the eluate obtained containing essentially the activated factor VII and the residual unactivated factor VII in the aqueous form, essentially free of other vitamin K-dependent factors, is then subjected to a viral inactivation stage, for example by the use of a TNBP/Tween mixture, while keeping to the relative proportions 0.3% of TNBP and 1% of Tween with respect to the total concentration of the solution in activated factor VII/factor VII, preferably for 6 hours at 24°C with stirring, and the inactivation mixture is then removed by adsorption on the same column with the same buffer solution which makes possible selective adsorption (NaCl 150 mM, trisodium citrate 4 mM and pH 6), with selective desorption with the same above-mentioned selective desorption buffer solution.

9. Process according to claim 8, characterized in that diafiltration is carried out against a buffer, preferably compatible with injection into man, such as a NaCl 100 mM, trisodium citrate 4 mM, lysine 3 g/l, pH 7 buffer.

10. Process according to claim 9, characterized in that concentration is carried out to a level of 5000 IU of activated factor VII in a 20 ml bottle and in that sterile filtration can be carried out by filtration through a sterilizing filter with a pore size of 0.2 micrometer and then lyophilization is optionally carried out.

## Patentansprüche

1. Verfahren zur Herstellung eines Konzentrats an hochreinem aktiven Faktor VII, das die Verwendung eines vom Kryopräzipitat befreiten Plasmas, vorzugsweise menschlichen Ursprungs, sowie mindestens einen Reinigungsschritt durch mindestens eine Chromatographie auf einem Anionenaustauscherzharz, sowie einen Aktivierungsschritt des Faktors VII umfaßt, dadurch gekennzeichnet, daß man zunächst die direkte Aktivierung des Faktors VII in dem vom Kryopräzipitat befreiten Plasma ohne Zufuhr von exogenen Proteinen durch die Verwendung von in dem Plasma unlöslichem Dextransulfat in Pulverform durchführt.

2. Verfahren nach Anspruch 1, dadurch gekennzeichnet, daß das Dextransulfat in Form von Kügelchen verwendet wird.

3. Verfahren nach einem der Ansprüche 1 bis 2, dadurch gekennzeichnet, daß der Schritt zur Aktivierung des Faktors VII vorzugsweise mit Dextransulfat in der Kälte unter kontollierter Temperatur, vorteilhafterweise im Bereich von 0 bis +4°C, durchgeführt wird.

4. Verfahren nach einem der Ansprüche 1 bis 3, dadurch gekennzeichnet, daß man nach dem Aktivierungsschritt eine DEAE-Anionenaustauschchromatographie des vom Kryopräzipitat befreiten, den aktiven Faktor VII sowie den nicht aktiven Faktor VII enthaltenden Plasmas so ausführt, daß alle Vitamin K-abhängigen Faktoren selektiv adsorbiert werden.

5. Verfahren nach Anspruch 4, dadurch gekennzeichnet, daß man eine nicht selektive Desorption aller auf der DEAE-Ionenaustauschersäule adsorbierten Vitamin K-abhängigen Faktoren durchführt.

6. Verfahren nach Anspruch 5, dadurch gekennzeichnet, daß das gewonnene Eluat, das die Vitamin K-abhängigen Faktoren enthält, erneut durch eine spezifische Anionenaustauschersäule vom quartären Aminotyp, vorzugsweise vom Typ Q-Sepharose Fast Flow® von PHARMACIA, das in Form von zu etwa 6% vernetzten Agarosekügelchen vorliegt und vorzugsweise einen kleinen $C_1$-$C_6$-Spacerarm enthält, so durchgeleitet wird, daß alle Vitamin K-abhängigen Faktoren selektiv adsorbiert werden.

7. Verfahren nach Anspruch 6, dadurch gekennzeichnet, daß man anschließend eine selektive Desorption des aktiven und nicht aktiven Faktors VII durchführt, indem eine Elution der Säule mit einer Pufferlösung zur selektiven Desorption mit der folgenden Zusammensetzung durchgeführt wird:

   - NaCl 250 mM
   - Tri-Na-citrat 4 mM
   - pH 6

8. Verfahren nach Anspruch 7, dadurch gekennzeichnet, daß das gewonnene Eluat, das im wesentlichen den aktiven Faktor VII und den restlichen nicht aktiven Faktor VII in wäßriger, im wesentlichen von anderen Vitamin K-abhängigen Faktoren befreiter Form enthält, einen weiteren Schritt zur Inaktivierung von Viren durchläuft, beispielsweise durch 6-stündige Verwendung einer Mischung von TNBP/Tween bei 24°C unter Rühren, wobei man einen relativen Anteil von 0,3 % TNBP und 1 % Tween im Verhältnis zur Gesamtkonzentration der Lösung von aktivem Faktor VII/Faktor VII einhält, wonach die Inaktivierungsmischung durch Adsorption auf der gleichen Säule mit der gleichen Pufferlösung beseitigt wird, wodurch die selektive Adsorption (NaCl 150 mM, Tri-Na-citrat 4 mM und pH 6) bei selektiver Desorption mit der gleichen selektiv desorbierenden Pufferlösung sichergestellt wird.

9. Verfahren nach Anspruch 8, dadurch gekennzeichnet, daß eine Diafiltration gegen eine Pufferlösung, die vorzugsweise bei Injektion in Menschen verträglich ist, wie etwa eine Pufferlösung aus 100 mM NaCl, 4 mM Tri-Na-citrat, 3g/l Lysin und pH 7, durchgeführt wird.

10. Verfahren nach anspruch 9, dadurch gekennzeichnet, daß eine Konzentration auf einen Wert von 5000 IU des aktiven Faktors VII pro 20 ml-Kolben vorgenommen wird, indem man durch einen Sterilfilter mit einer Porengröße von 0,2 µm sterilfiltriert und anschließend eine Lyophilisierung durchführt.